# EUROPEAN PATENT SPECIFICATION

(11) **EP 2 152 364 B1**
(45) Date of publication and mention of the grant of the patent: **22.08.2012**
(21) Application number: 08715430.8
(22) Date of filing: 07.03.2008
(51) Int. Cl.: A61N 5/06

(54) **APPARATUS FOR LIGHT THERAPY**
LICHTTHERAPIEGERÄT
APPAREIL DE LUMINOTHERAPIE

(30) Priority: 07.03.2007 CZ 20070177
(43) Date of publication of application: 17.02.2010
(73) Proprietor: Voves, Vladimir, 395 26 Lukavec (CZ)
(72) Inventor: Voves, Vladimir, 395 26 Lukavec (CZ)
(74) Representative: Fischer, Michael
(86) International application number: PCT/CZ2008/000030
(87) International publication number: WO 2008/106905

(56) References cited:
- EP-A- 0 311 125
- WO-A-00/30714
- WO-A-84/03049
- WO-A-96/04959
- DE-A1- 10 041 855
- US-A- 4 686 986

## Description

### Technical field

The invention relates to an apparatus for light therapy, including an outer tube case with a first end and a second end, an inner angular tube case embedded in the outer case and including an input end with a light source and a reflector, the input end extending to the first end of the outer case, a middle section with Brewster polarizer parallel glass plates forming a part of the wall of the middle section, and the output end extending into the second end of the outer case, and a cooling fan embedded in the outer case for the cooling of the reflector and of the polarization plates of the inner case.

### State of the Art

The objective of light-therapy apparatuses is to promote activity of cells and healing processes on the body surface. Cell activation and stimulation of biological processes are achieved using linearly polarized light, which is emitted by light rays transmitted from a light source and streamlined by a reflector into a light beam, by reflection from reflection surfaces of Brewster polarizer parallel glass plates, which are arranged in a reflex angle towards direction of the light beam. If the light-source output is rather high, both the reflector and Brewster polarizer glass plates heat up heavily, releasing loads of heat, which have to be vented by a stream of air generated by a fan.

According to the document EP 0 311 125, the Brewster polarizer consists of an angular tube-case, which includes an input end with the light source and rectifying reflector, an angular middle section with parallel glass plates, which form part of the tube-case wall, and an output end with an optical filter. The tube-case, being the inner case, is embedded in the middle section of the outer case. A fan is arranged in the first end of the outer case, and the output end of the inner tube-case extends into the open second end of the outer case. The inner tube-case is embedded in the outer case with a peripheral annular gap. The fan sucks air from the space between the inner case and outer case, creating undepressure due to which cooling air circulates in the annular gap between the output end of the inner tube-case and the second end of the outer case to the space between the outer case wall and inner tube-case, flowing first along the surface of the inner tube-case, then along the Brewster polarizer glass plates, which form part of the inner tube-case wall, and finally, along the light-source reflector. Sucked-in air is pushed by the fan out of the outer case through the hole created in the first end of the outer case.

The described direction of the air-flow is advantageous because it flows well round Brewster polarizer glass plates and cools them. To cool the reflector extending from the input end of the inner case, however, there should be a small, circumferential slot to increase significantly the speed of the air-flow and take away more quickly the heat from the reflector. The measure is not, however, quite sufficient to cool the reflector. The given direction of the air-flow is disadvantageous also because the large quantities of air sucked into the annular gap between the second end of the outer case and the output end of the inner case pull along loads of biological dirt from the part of the human body being treated. The biological dirt covers and deposits in an undesirable way the inner installation of the apparatus. The phenomenon is undesirable especially because the dirt is of biological origin and can be a source of various infections. Direction of the flow of cooling air can be reversed, and the fan can suck in cooling air through the hole in the first end of the outer case, push out cooling air to the annular gap between the inner case and outer case, and the heated cooling air can go out from the outer case through the annular gap between the output end of the inner tube-case and the second end of the outer case. The reflector is cooled with higher efficiency because it is located against the direction of cooling air flowing from the fan; the higher cooling efficiency is, however, offset by the flow of high quantities of cooling air to the part of the human body being treated. The existing insufficient cooling capacities for the reflector and inner body prevent attempts to increase performance of the apparatus for light therapy so as to improve efficiency of the curing effect.

The aim of the present invention is to eliminate disadvantages of the existing state of the art and offer an apparatus for light therapy with improved cooling, with higher light performance at the output, with a simple design, with better control, and with improved curing effects; an apparatus, which is more hygienic and does not foul with dirt or dust.

### Summary of the Invention

The apparatus according to the invention eliminates into a considerable extent the disadvantages of the existing state of the art and meets the aim of the invention. The apparatus according to the invention consists of an outer tube-case with the first end and second end, an inner angular tube-case, which is embedded in the outer case and includes the input end with a light source and reflector, extending to the first end of the outer case, a middle section with Brewster polarizer parallel glass plates, which form part of the wall of the middle section, and the output end extending into to the outer case second end, and a cooling fan embedded in the outer case for the cooling of the reflector and inner case polarization plates according to the invention consisting in that a hole is arranged in the outer case wall in the transition section between the first end and second end of the outer case. The hole is interconnected with the open first end of the outer case to allow passage of cooling air. A sealing partition with a slot and adjustable flow cross-section can be advantageously arranged between the second end of the outer case and the output end of the inner case. The cooling fan can be advantageously arranged between the hole in the outer case first end and the inner case input end. According to the alternative design of the invention, a ventilating duct can be advantageously connected to the hole, forming a tube-like body in the form of star with the outer case first end and outer case second end, where the cooling fan is arranged in the ventilation duct. The hole can be advantageously arranged opposite the Brewster polarizer plates.

Cooling air does not flow mainly through the second end of the outer case, but mainly or only through the hole in the partition wall in the outer case. In this way the site being treated is affected much less by cooling air. It is possible to let flow only small amounts of cooling air through the annular hole between the output end of the inner case and the second end of the inner case; cooling air flowing there can be advantageously controlled, or the entry to that annular space can be closed totally. In this way it is possible to control the amount of air flowing to the site being treated or totally close the inflow of air to the treated site. Small amounts of air flowing to the treated site can improve the curing process without adverse effects. Since small amounts of air flow through the second end of the outer casing, the apparatus does not foul with sucked in dirt. The apparatus for light therapy according to the invention makes it possible to select direction of the flow of cooling air. Cooling air can flow both in the direction of the light beam, cooling intensively the reflector, and against the direction of the light beam, cooling intensively the Brewster polarizer plates. The change in the air flow can influence positively the curing effect. The apparatus for light therapy according to the invention features improved cooling because the outer case is fitted with larger flow cross-sections, providing for the flow of higher quantities of air. With improved cooling, the apparatus for light therapy according to the invention can have higher light performance. The apparatus for light therapy according to the invention features a simple design of the outer case, and it is easy to assemble. The apparatus for light therapy according to the invention can be easily fixed to a stand and positioned together with the stand in a suitable distance from and position towards the part of the human body being treated.

### Summary of Figures on the Drawings

The apparatus for light therapy according to the invention is illustrated on figures where Fig. 1 depicts a longitudinal section of the apparatus with the fan arranged in the first end of the outer case; Fig. 2 depicts a longitudinal section of the apparatus with the fan arranged in the ventilation duct; and Fig. 3 is a perspective top-view of the apparatus in a partial section.

### Exemplary Embodiment of the Invention

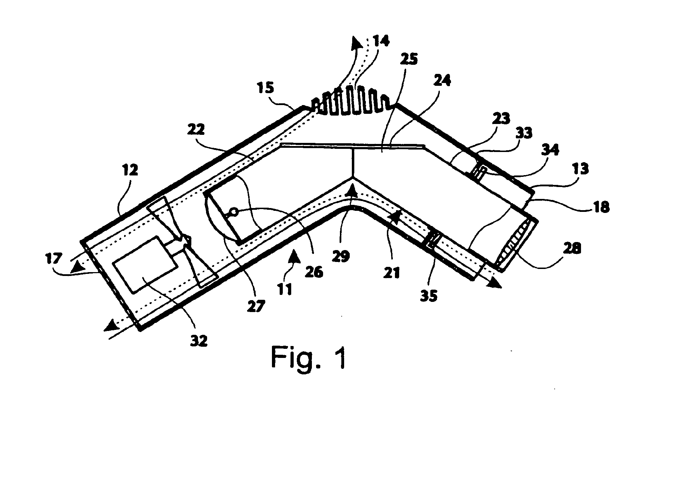

As indicated by Fig. 1, the outer case 11 of the apparatus for light therapy consists of the tube-like first end 12, which is followed by the angular transition middle section 19, which merges into the tube-like second end 13. Both the first end 12 and second end 13 of the outer case 11 are exemplary open. In the outer case 11 there is a Brewster polarizer consisting of the inner case 21 with the tube-like input end 22, which merges into the angular middle section 29 with a part of the wall 25 consisting of a set of parallel polarization glass plates 24. The angular middle section 29 merges into the tube-like output end 23, which is terminated with the glass light filter 28. The inner case 21 can be arranged in the outer case 11 in such a way that the axis of the output end 22 of the inner case 21 and the axis of the first end 12 of the outer case 11, or the output end 23 of the inner case 21 and the second end 13 of the inner case 11 advantageously merge, and the output end 23 of the inner case 21 partially extends out of the second end 13 of the outer case 11. Between the output end 23 of the inner case 21 and the second end 13 of the outer case 11, there is the sealing partition 33 arranged, and it can be advantageously fitted with the slot 32 the flow cross-section of which can be changed by the turning ring 35. The turning ring 35 is exemplary arranged in the tube-like inner case 21 and its perimeter is of various radiuses. By turning the ring 35, it is possible to change the size of the flow cross-section of the slot 34. In the orifice of the input end 22 there is the reflector 27 with the front-end light source 26, which lies approximately in the focus of the reflector 27 to streamline emitted light rays into a light-beam in the axis of the input end 22. After the light-beam impinges reflection surfaces of the parallel polarization glass plates 24, light rays polarize by refraction and go out through the output end 23 towards the site on the human body being treated. Between the orifice of the outer case first end 12 and the reflector there is the fan 32 arranged. The position of the plastic inner case 21 is advantageously fixed in the plastic outer case 11 by means of interlocking surface stumps, which are not illustrated. The fan 32 is also arranged in the surface stumps of the outer case first end 12. In the transition section between the first end 12 and second end 13 there is the hole 14 arranged in the wall 15 of the outer case 11 to allow for passage of cooling air. The flow cross-section of the hole 14 is larger than the flow cross-section of the annular space between the output end 23 of the inner case 21 and the second end 13 of the outer case 11. The smallest flow cross-section of the second end 23 of the outer case 11 is advantageously smaller than the smallest flow cross-section of the hole 14. Between the output end 23 of the inner case 21 and the second end 13 of the outer case 11 there is the sealing partition 33 with the slot 34 the flow cross-section of which can be controlled. The fan 32 sucks in cooling air through the front hole in the first end 12 of the outer case along the marked continuous lines; cooling air going out of the fan 32 flows advantageously round the reflector 27, then through the space between the input end 22 of the inner case 21 and the first end 12 of the outer case to leave through the hole 14 in the wall 15. Depending on the size of the flow cross-section between the output end 23 of the inner case 21 and the second end 13 of the outer case 11, a portion of cooling air can leave also through the second end 13 of the outer case 11 towards the treated site of the human body. The amount of cooling air leaving through the second end 13 of the outer case 11 can be controlled by closing or opening the slot 34 in the sealing partition 33. As indicated by the marked dash lines, direction of the cooling-air flow according to Fig. 1 can be reversed. Cooling air can be sucked in through the hole 14 in the wall 15 and, optionally, small amounts of cooling air can also be sucked in through the open second end 13 of the outer case 11 or through the slot 34 in the sealing partition 33. Having passed through the fan 32, cooling air leaves through the open first end 12 of the outer case 11. Brewster polarization plates 24 can be advantageously arranged opposite the hole 14 so that they are cooled heavily by the flow of incoming air during the given flow direction. Direction of the air flow can be advantageously changed.

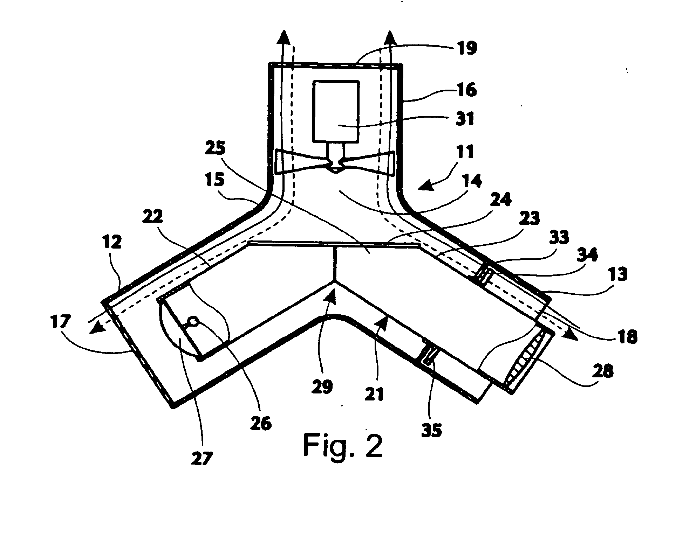

Advantageous design of the invention according to Fig. 2 includes the ventilation duct 16 connected to the wall 15 and hole 14 and forming, together with the first end 12 and second end 13 of the outer case 11, a tube-like, star-shaped body. The fan 31 can be advantageously seated in the ventilation duct 16, creating undepressure in the ventilation duct 16, due to which cooling air is sucked in along the marked continuous lines through the open first end 12 of the outer case 11; then, cooling air flows in the space between the input end 22 of the inner case 21 and first end 12 of the outer case 11 to enter the hole 14 in the wall 15. Simultaneously, a portion of cooling air can be sucked in also through the second end 13 of the outer case 11. The smallest flow cross-section between the output end 23 of the inner case 21 and the second end 13 of the outer case 11 is advantageously smaller than the smallest flow cross-section of the first end 12 of the outer case 11. Similarly to Fig. 1, direction of the cooling-air flow according to Fig. 2 can be reversed, as indicated by dash direction lines, and optionally, small amounts of cooling air can also be sucked in through the open second end 13 of the outer case 11 or through the slot 34 in the sealing partition 33. Brewster polarization plates 24 can be advantageously arranged opposite the hole 14 so that they are cooled heavily by the flow of incoming air during the given flow direction. Direction of the air flow can be advantageously changed.

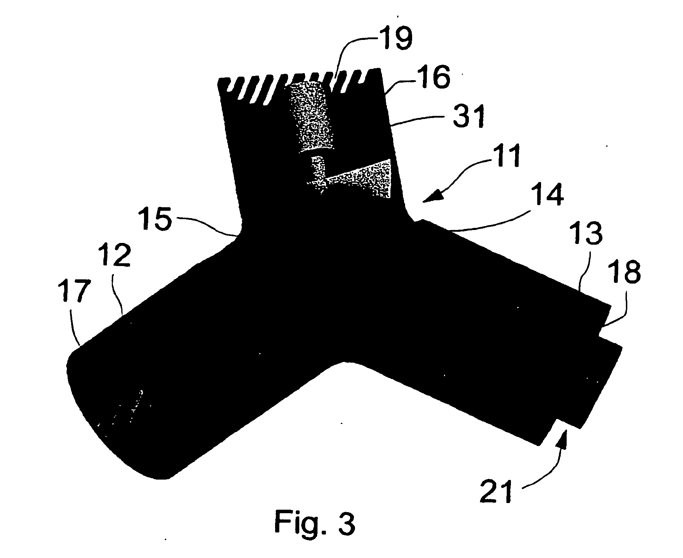

The outer case 11 of the apparatus for light therapy can advantageously be, according to Fig. 3, in the form of a star-shaped, tube-like body, which is advantageous for the seating of crucial parts, i.e. the inner case 21 and the fan 31. Fig. 3 indicates the hole 17 in the orifice of the first end 12, the hole 18 in the orifice of the second end 13 of the outer case 11, and the hole 19 in the orifice of the ventilation duct 16. Surface stumps 36 can be used to seat and fix the outer case 11 to a stand, which is not illustrated.

## Claims

1. Apparatus for light therapy, including an outer tube case (11) with a first end (12) and a second end (13), an inner angular tube case (21) embedded in the outer case (11) and including an input end (22) with a light source (26) and a reflector (27), the input end (22) extending to the first end (12) of the outer case (11), a middle section (29) with Brewster polarizer parallel glass plates (24) forming a part of the wall (25) of the middle section (29), and the output end (23) extending into the second end (13) of the outer case (11), and a cooling fan (31, 32) embedded in the outer case (11) for the cooling of the reflector and of the polarization plates (24) of the inner case (21), in
**characterised in that**
a transition section of the wall (15) of the outer case (11) between the first end (12) and the second end (13) of the outer case (11) is provided with a hole (14) interconnected with the open first end (12) of the outer case (11) for passage of cooling air.

2. Apparatus for light therapy according to the claim 1,
wherein
a sealing partition (33) with a slot (34) having adjustable flow cross-section is arranged between the second end (13) of the outer case (11) and the output end (23) of the inner case (21).

3. Apparatus for light therapy according to the claims 1 or 2, wherein
a cooling fan (32) is arranged between a hole (17) of the first end (12) of the outer case (11) and the inlet end (22) of the inner case (21).

4. Apparatus for light therapy according to the claims 1 or 2, wherein
a ventilating duct (16) is connected to the hole (14), the ventilating duct (16) forming with the first end (12) of the outer case (11) and with the second end (13) of the outer case (11) a tube-like body in the form of star, whereas a cooling fan (31) is arranged in the ventilation duct (16).

5. Apparatus for light therapy according to the claims 1 or 3, wherein
the hole (14) is arranged opposite the Brewster polarizer plates (24).

## Patentansprüche

1. Gerät zur Lichttherapie, beinhaltend eine äußere röhrenförmige Hülle (11), mit einem ersten Ende (12) und einem zweiten Ende (13),
eine innere Hülle (21) von gebrochener Rohrform, gelagert in der äußeren Hülle (11) und beinhaltend
. ein Eingangsende (22) mit einer Lichtquelle (26) und mit einem Reflektor (27), wobei das Eingangsende (22) in das erste Ende (12) der äußeren Hülle (11) hineinreicht,
. einen Mittelteil (29) mit parallelen gläsernen Polarisierungsplatten (24) eines Brewster Polarisators, die einen Teil der Wand (25) des Mittelteils (29) darstellen, und ein
. Ausgangsende (23) das in das zweite Ende (13) der äußeren Hülle (11) hineinreicht,
und
Kühlventilator (31, 32), gelagert in der äußeren Hülle (11) zur Kühlung des Reflektors (27) und der Polarisierungsplatten (24) der inneren Hülle (21),
gekennzeichnet t **dadurch**, dass der Übergangsteil der Wand (15) der äußeren Hülle (11) zwischen dem ersten Ende (12) und zweiten Ende (13) der äußeren Hülle (11) mit einer Öffnung (14) versehen ist, die für den Durchlauf der Kühlungsluft mit dem offenen ersten Ende (12) der äußeren Hülle (11) verbunden ist.

2. Gerät zur Lichttherapie laut Anspruch 1,
wo zwischen dem zweiten Ende (13) der äußeren Hülle (11) und dem Ausgangsende (23) der inneren Hülle (21) eine Dichtungswand (33) mit Spalte (34) mit verstellbarem Durchlassquerschnitt angeordnet ist.

3. Gerät zur Lichttherapie laut Anspruch 1 oder 2,
wo der Kühlventilator (32) zwischen der Öffnung (17) des ersten Endes (12) der äußeren Hülle (11) und dem Eingangsende (22) der inneren Hülle (21) angeordnet ist.

4. Gerät zur Lichttherapie laut Anspruch 1 oder 2,
wo der Ventilationskanal (16) an die Öffnung (14) angeschlossen ist, der Ventilationskanal (16) mit dem ersten Ende (12) der äußeren Hülle (11) und dem zweiten Ende (13) der äußeren Hülle (11) einen Rohrkorpus in Sternform darstellt, wobei im Ventilationskanal (16) ein Kühlventilator (31) installiert ist.

5. Gerät zur Lichttherapie laut Anspruch 1 oder 3,
wo die Öffnung (14) gegenüber den Polarisierungsplatten (24) des Brewster Polarisators angeordnet ist.

## Revendications

1. Appareil de luminothérapie, comprenant un fourreau (11) extérieur de forme tubulaire ayant une première extrémité (12) et une seconde extrémité (13),
un fourreau (21) intérieur de forme tubulaire brisée, placé dans le fourreau (11) extérieur et comprenant
. une extrémité d'entrée (22) munie d'une source de lumière (26) et d'un réflecteur (27), en sachant que l'extrémité d'entrée (22) pénètre dans la première extrémité (12) du fourreau (11) extérieur,
. une partie centrale (29) contenant les lames polarisantes (24) du polariseur de Brewster, parallèles, en verre, qui forment une partie de la cloison (25) de la partie centrale (29), et
. une extrémité de sortie (23) pénétrant dans la seconde extrémité (13) du fourreau (11) extérieur,
et
un ventilateur de refroidissement (31, 32), placé dans le fourreau (11) extérieur, servant à refroidir le réflecteur (27) et les lames polarisantes (24) du fourreau (21) intérieur,
**caractérisé en ce que** la partie transitoire d'une aire (15) du fourreau (11) extérieur située entre la première extrémité (12) et la seconde extrémité (13) du fourreau (11) extérieur est munie d'un orifice (14) raccordé avec la première extrémité (12) ouverte du fourreau (11) extérieur, ceci pour le passage de l'air de refroidissement.

2. Appareil de luminothérapie en fonction de la revendication 1,
ou entre la seconde extrémité (13) du fourreau (11) extérieur et l'extrémité de sortie (23) du fourreau (21) intérieur se trouve une paroi (33) d'étanchéité munie d'une fente (34) ayant une section de débit réglable.

3. Appareil de luminothérapie en fonction des revendications 1 ou 2,
ou le ventilateur (32) de refroidissement est installé entre l'orifice (17) de la première extrémité (12) du fourreau (11) extérieur et l'extrémité d'entrée (22) du fourreau (21) intérieur).

4. Appareil de luminothérapie en fonction des revendications 1 ou 2,
ou le canal de ventilation (16) est raccordé à l'orifice (14), le canal de ventilation (16) forme, avec la première extrémité (12) du fourreau (11) extérieur et la seconde extrémité (13) du fourreau (11) extérieur, un corps tubulaire en forme d'étoile, en sachant que le ventilateur (31) de refroidissement se trouve dans le canal (16) de ventilation.

5. Appareil de luminothérapie en fonction des revendications 1 ou 3,
ou l'orifice (14) se trouve en face des lames polarisantes (24) du polariseur de Brewster.
